# EUROPEAN PATENT APPLICATION

(11) **EP 4 692 333 A1**
(43) Date of publication of application: **11.02.2026**
(21) Application number: 24797180.7
(22) Date of filing: 26.04.2024
(51) Int. Cl.: C12N 5/10, C12N 15/88

(54) **CELL CAPSULE, CELL POPULATION, CELL PRODUCT, CELL PREPARATION, AND METHOD FOR PRODUCING CELL POPULATION**

(30) Priority: 28.04.2023 JP 2023075061
(71) Applicant: Cellfiber Co., Ltd., Tokyo 135-0031 (JP); Mie University, Tsu-shi, Mie 514-8507 (JP)
(72) Inventor: IKEDA Kazuhiro, Tokyo 135-0031 (JP); KANEIWA Tomoyuki, Tokyo 135-0031 (JP); MIWA Hiroshi, Tsu-shi, Mie 514-8507 (JP)
(74) Representative: Isarpatent
(86) International application number: PCT/JP2024/016444
(87) International publication number: WO 2024/225435

(57) **Abstract**

Provided is a method for producing a method for producing a cell population capable of providing an improved method for causing a cell take up a substance to be introduced. The method for producing a cell population comprises: covering cells and a delivery substance with a semipermeable membrane (14) to form a cell capsule (10); and culturing the cells in the cell capsule (10).

## Description

### Technical field

The present invention relates to a cell capsule, a cell population, a cell product, a cell preparation, and a method for producing a cell population.

### Background Art

Techniques for introducing introduction substances such as nucleic acids and proteins into cells are known. One of such techniques is gene introduction. As one main method of gene introduction, a method using a virus or viral vector is known. Examples of viruses used for gene introduction include retroviruses, lentiviruses, adenoviruses, and adeno-associated viruses. These viruses infect cells through specific receptors present on the cell surface. The viruses introduce genes into cells by utilizing their inherent cell entry mechanisms.

Patent Literature 1 below describes that, during gene introduction using a retrovirus, activated T cells are cultured on a plate coated with RetroNectin.

### Citation List

### Patent Literature

Patent Literature 1: WO 2019/075395 A1

### Summary

In the gene introduction described in Patent Literature 1, for example, reagents that enhance gene transfer efficiency, such as RetroNectin, are used. However, since RetroNectin is a coating agent, it is necessary to immobilize RetroNectin on a plate or dish used for culturing cells together with a virus. For this reason, centrifugation operation and long-term incubation are required, thereby increasing the number of production processes. In addition, because a surface coated with RetroNectin is required, scale-up of the culture is also difficult.

Accordingly, there remains room for improvement in methods for incorporating introduction substances such as nucleic acids or proteins into cells, materials used therefor, cell populations into which introduction substances have been introduced, structures including the same, and the like.

In one aspect, a method for producing a cell population, comprises: covering cells and a delivery substance with a semipermeable membrane to form a cell capsule; and culturing the cells in the cell capsule.

In one aspect, a cell population is one obtained by the method for producing the cell population as described above.

In one aspect, a cell preparation is one obtained from the cell population as described above.

In one aspect, a cell product is one obtained from the cell population as described above.

In one aspect, a cell capsule comprises: a core containing cells and an introduction substance; and a semipermeable membrane covering the core.

### Brief Description of Drawings

Fig. 1 is a schematic perspective view of a cell capsule according to one embodiment.
Fig. 2 is a schematic cross-sectional view of the cell capsule according to one embodiment.
Fig. 3 is a schematic view of a cell capsule according to another embodiment.
Fig. 4 is a view for explaining one example of a method for forming a cell capsule.
Fig. 5 is a view for explaining another example of a method for forming a cell capsule.
Fig. 6 is a view showing microscopic photographs of cell capsules from Day 4 to Day 11 in Example 1.
Fig. 7 is a view showing microscopic photographs of cell capsules from Day 4 to Day 11 in Reference Example 1-1.
Fig. 8 is a view showing analysis results of cell populations by flow cytometry in Example 1 and Reference Examples 1-1 and 1-2.
Fig. 9 is a view showing analysis results of cell populations by flow cytometry in Example 2 and Reference Examples 2-1 and 2-2.
Fig. 10 is a view showing results of measurement of the amount of cytokine (IFN-γ) produced from the cell populations in Example 2 and Reference Example 2-1.
Fig. 11 is a view showing analysis results of cell populations by flow cytometry in Example 3 and Reference Examples 3-1 and 3-2.
Fig. 12 is a view showing analysis results of cell populations in Example 4 and Reference Examples 4-1 and 4-2.

### Description of Embodiments

Hereinafter, embodiments will be described with reference to the drawings. In the following drawings, the same or similar parts are denoted by the same or similar reference numerals. However, it should be noted that the drawings are schematic, and ratios of dimensions and the like may be different from actual ones.

### [Cell capsule and cell population]

A cell capsule according to one embodiment includes: a core containing cells and an introduction substance; and a semipermeable membrane covering the core. The core may contain one or a plurality of cells. Preferably, the core contains a plurality of cells.

The introduction substance may be in a state of having been introduced into the cells. After carrying out a method of introducing the introduction substance into the cells as described below, the introduction substance will be in a state of having been introduced into a plurality of cells or many cells. The introduction substance is introduced into the cells by using a delivery substance described below.

The semipermeable membrane may be a membrane permeable to oxygen and/or nutrients. The semipermeable membrane only has to cover the cells, and may have, for example, a substantially tubular shape or a substantially spherical shell shape. The substantially spherical shell shape does not necessarily mean a perfect spherical shell, and may also include deformed ones such as an ellipsoid.

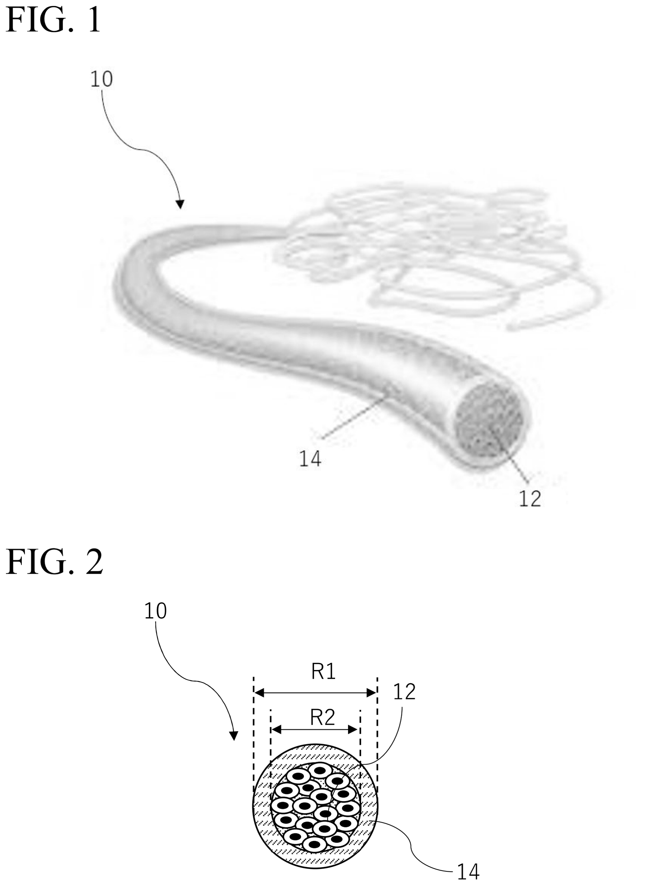

Fig. 1 is a schematic view showing a configuration of a cell capsule according to one aspect. Fig. 2 is a schematic cross-sectional view of the cell capsule shown in Fig. 1. In the aspect shown in Figs. 1 and 2, a semipermeable membrane 14 has a substantially tubular shape. Specifically, the cell capsule may include the tubular semipermeable membrane 14 and a core 12 covered with the semipermeable membrane 14. Cells are contained in the core 12. The semipermeable membrane 14 surrounds the periphery of the core 12. More specifically, the semipermeable membrane 14 surrounds the core 12 in a cross section perpendicular to the direction in which the semipermeable membrane 14 extends. The tubular semipermeable membrane 14 extends longitudinally, and the core 12 extends in a string-like manner inside the tubular semipermeable membrane 14.

The length of the semipermeable membrane 14 may be, for example, 5 cm or more, more preferably 10 cm or more, and still more preferably 20 cm or more. The longer the length of the semipermeable membrane 14, the more cells can be contained in one cell capsule. The length of the semipermeable membrane 14 is not particularly limited, but may be, for example, 10 m or less.

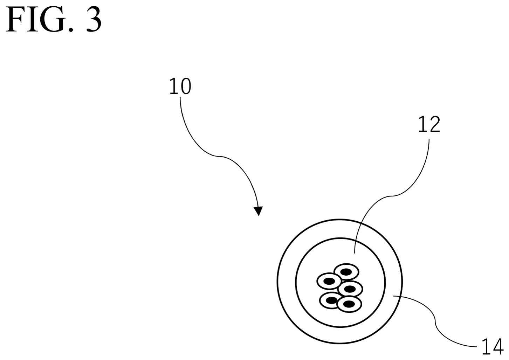

Fig. 3 is a schematic view showing a configuration of a cell capsule according to another aspect. In the aspect shown in Fig. 3, a cell capsule 10 has a substantially spherical shape. A semipermeable membrane 14 has a substantially spherical shell shape. Specifically, the cell capsule may contain the substantially spherical shell-shaped semipermeable membrane 14 and a core 12 covered with the semipermeable membrane 14. The core 12 is substantially spherical, and the semipermeable membrane 14 surrounds the entire core 12. Cells are contained in the core 12. The cell capsule according to the aspect shown in Fig. 3 may be considered a shortened version in length of the cell capsule according to the aspect shown in Figs. 1 and 2.

Figs. 1 to 3 show preferred examples of shapes of the cell capsule, but the shapes of the cell capsule are not limited thereto.

In the cell capsule according to any aspect, the outer diameter of the semipermeable membrane is not particularly limited, but may be, for example, in a range of 100 µm to 5000 µm, preferably in a range of 150 µm to 3000 µm, and more preferably in a range of 200 µm to 1000 µm. When the semipermeable membrane has a substantially tubular shape, the outer diameter of the semipermeable membrane may be defined by an outer diameter of the semipermeable membrane in a cross section perpendicular to the direction in which the cell capsule extends (see reference numeral R1 in Fig. 2). When the semipermeable membrane has a substantially spherical shell shape, the outer diameter of the semipermeable membrane may be defined by a minimum outer diameter of the semipermeable membrane in a cross section passing through a center (center of gravity) of the cell capsule.

The inner diameter of the semipermeable membrane is not particularly limited, but may be, for example, in a range of 20 µm to 4000 µm, preferably in a range of 60 µm to 2000 µm, and more preferably in a range of 100 µm to 1000 µm. When the semipermeable membrane has a substantially tubular shape, the inner diameter of the semipermeable membrane may be defined by an inner diameter of the semipermeable membrane in a cross section perpendicular to the direction in which the cell capsule extends (see reference numeral R2 in Fig. 2). When the semipermeable membrane has a substantially spherical shell shape, the inner diameter of the semipermeable membrane may be defined by a minimum inner diameter of the semipermeable membrane in a cross section passing through a center (center of gravity) of the cell capsule. When the inner diameter of the semipermeable membrane becomes smaller, a volume inside the semipermeable membrane becomes smaller, and the cells and the delivery substance can be locally densified. This makes it possible to improve efficiency of incorporating the introduction substance into the cells by the delivery substance containing the introduction substance. The inner and outer diameters described above may be measured, for example, by a phase-contrast optical microscope.

The thickness of the semipermeable membrane is not particularly limited, but may be, for example, 5 µm or more, preferably 20 µm or more, and more preferably 50 µm or more. The thicker the semipermeable membrane, the more easily the delivery substance is considered to be confined inside the semipermeable membrane. The thickness of the semipermeable membrane may be, for example, 1000 µm or less, preferably 300 µm or less, and more preferably 100 µm or less. This makes it easier for medium components to permeate into the semipermeable membrane.

The semipermeable membrane preferably contains a hydrogel. The hydrogel is obtained by gelling a hydrogel precursor. The hydrogel preferably has sufficient permeability to cell culture medium components. On the other hand, the hydrogel preferably has low permeability to the delivery substance or is impermeable to the delivery substance. This allows many delivery substances in the cell capsule to be confined inside the semipermeable membrane. From this perspective, it is desirable that permeability of the delivery substance to the semipermeable membrane 14 be lower than permeability of the cell culture medium components to the semipermeable membrane 14.

The hydrogel may include at least one selected from the group consisting of alginate gels, Matrigel, collagen gels, chitosan gels, gelatin, peptide gels, laminin gels, agarose gels, nanocellulose, methylcellulose, dextran, pectin, gellan gum, xanthan gum, guar gum, carrageenan, glucomannan, and fibrin gels.

More preferably, the hydrogel contains a gel mainly composed of an alginate gel, that is, contains an alginate gel. In this case, the hydrogel precursor may be a solution mainly composed of an alginate solution. The alginate gel can be formed by crosslinking an alginate solution, as an alginate derivative, with a divalent metal ion. The alginate gel can be suitably used for culturing cells or cell masses in a state where the cells or the cell masses are enclosed, and for confining many delivery substances.

The core may be, for example, a liquid or sol having fluidity, such as a cell suspension. The core may contain, for example, one selected from the group consisting of extracellular matrix, physiological saline, Ringer's solution, medium, culture supernatants, and buffers, or a mixture thereof.

The core may contain a thickener. The thickener may contain, for example, one selected from the group consisting of collagen solutions, collagen gels, gelatin, Matrigel, chitosan gels, alginate solutions, alginate gels, peptide gels, laminin, agarose, nanocellulose, methylcellulose, hyaluronic acid, proteoglycan, elastin, pullulan, dextran, dextrin, pectin, gellan gum, xanthan gum, guar gum, carrageenan, and glucomannan, or a mixture thereof.

The core may contain various growth factors suitable for culturing cells, maintaining or proliferating cells, expressing functions of cells, or the like. Such growth factors may include, for example, at least one selected from the group consisting of epidermal growth factor (EGF), platelet-derived growth factor (PDGF), transforming growth factor (TGF), insulin-like growth factor (IGF), fibroblast growth factor (FGF), nerve growth factor (NGF), vascular endothelial growth factor (VEGF), hepatocyte growth factor (HGF), interferons, interleukins, tumor necrosis factor-α (TNF-α), immunoglobulins, and chemokines.

The introduction substance is not particularly limited as long as it is a substance that is incorporated into cells. The introduction substance may contain, for example, at least one selected from the group consisting of nucleic acids, proteins, nanoparticles, and drugs. Here, the introduction substance may be a substance that has been introduced into a cell membrane. The introduction substance may be contained in a delivery substance described below, and may be incorporated into the cells by the delivery substance.

The size of the introduction substance, that is, the maximum diameter, may be, for example, 1000 nm or less, preferably 500 nm or less, and more preferably 200 nm or less. The size of the introduction substance, that is, the maximum diameter, may be, for example, 1 nm or more, preferably 10 nm or more, and more preferably 50 nm or more. The size of the introduction substance may be within a range obtained by arbitrarily combining the above upper limit and lower limit values.

The molecular weight of the introduction substance may be, for example, 1 kDa or more, preferably 10 kDa or more, and more preferably 100 kDa or more. The molecular weight of the introduction substance may be, for example, 10,000,000 kDa or less, preferably 1,000,000 kDa or less, and more preferably 500,000 kDa or less. The molecular weight of the introduction substance may be within a range obtained by arbitrarily combining the above upper limit and lower limit values.

The introduction substance may be, for example, a genetic material. In this case, the introduction substance may be introduced into the cells by, for example, a virus or a viral vector. The genetic material may include, for example, a nucleic acid encoding at least one selected from the group consisting of reporter proteins, leukocyte antigens, growth factors, antibodies, and siRNA. The reporter protein may include at least one selected from the group consisting of chimeric antigen receptors (CARs), TCRs, and GFPs.

The core may contain a delivery substance for delivering the introduction substance into the cells. The delivery substance may contain at least one selected from the group consisting of viruses, viral vectors, vaccines, extracellular vesicles, and liposomes, or a complex of these with the introduction substance. The vaccine may be, for example, a virus-like particle and/or an inactivated virus-derived component. Preferably, the delivery substance may be capable of binding to a receptor on the surface of the cells.

When the delivery substance is a virus or a viral vector, the virus or viral vector may include at least one selected from the group consisting of retroviruses, lentiviruses, adenoviruses, adeno-associated viruses, herpesviruses, and tobacco mosaic viruses. The virus or viral vector may also be a hybrid virus having functions of a plurality of these viruses.

A viral vector is a virus that infects target cells and contains an introduction substance to be introduced into the cells. Such an introduction substance may contain, for example, a nucleic acid encoding at least one selected from the group consisting of receptor proteins, membrane proteins, transcription factors, cytokines, reporter proteins, leukocyte antigens, growth factors, antibodies, and siRNA. The receptor protein is not particularly limited, but may be, for example, chimeric antigen receptor (CAR) or TCR. The reporter protein is not particularly limited, but may be, for example, GFP.

The type of the cells contained in the core is not particularly limited as long as it is a target cell into which the introduction substance is to be introduced and/or a cell into which the introduction substance has been introduced. The cells may be, for example, bacteria, microorganisms, plant cells, and/or animal cells. The animal cells are preferably mammalian cells, and more preferably human cells.

Preferably, the cells may be various stem cells having pluripotency, ES cells or iPS cells having totipotency, stem cells having pluripotency, or precursor cells having oligopotency or unipotency. Examples of various stem cells having totipotency or pluripotency include iPS cells, ES cells, mesenchymal stem cells, neural stem cells, hepatic stem cells, skin stem cells, pancreatic stem cells, and hematopoietic stem cells. Examples of stem cells or precursor cells having oligopotency or unipotency include muscle stem cells, germline stem cells, respiratory precursor cells, digestive precursor cells, cardiac precursor cells, lymphocyte precursor cells, and hematopoietic precursor cells.

Alternatively, the cells may include at least one selected from the group consisting of, for example, muscle cells such as skeletal muscle cells, smooth muscle cells, and cardiomyocytes, neural cells such as cerebral cortical cells, fibroblasts, epithelial cells, endothelial cells, adipocytes, osteoblasts, chondrocytes, macrophages, dendritic cells, hepatocytes, hepatic stellate cells, pancreatic β-cells, keratinocytes, renal cells, renal tubular cells, hair matrix cells, corneal endothelial cells, photoreceptor cells, pigment epithelial cells, goblet cells, respiratory cells, blood cells, T cells, NK cells, and dendritic cells. These cells may also be cells prepared by inducing differentiation from cells having totipotency or pluripotency.

More preferably, the cells may be at least one selected from blood cells, T cells, NK cells, dendritic cells, hematopoietic stem cells, lymphocyte precursor cells, and hematopoietic precursor cells. These cells are suitable for the purpose of gene therapy.

At least a part of the above composition contained in the core may be contained in the cell capsule from the beginning. Alternatively and/or additionally, at least a part of the above composition contained in the core may enter the inside of the cell capsule from a medium during culturing of the cells in the cell capsule as described below, and may also be produced from the cells during the culturing.

In one example, the cells contained in the core may be, for example, T cells and/or NK cells expressing a chimeric antigen receptor, T cells expressing a T cell receptor (TCR), or stem cells, precursor cells, and/or differentiated cells into which other genes have been introduced.

The cell capsule may contain a plurality of cells. In the present embodiment, a cell population may be composed of a plurality of cells contained in the cell capsule. Accordingly, the type of cells constituting the cell population may be the same as the type of cells contained in the core described above.

In a specific example, when the cell capsule has a long string-like shape as shown in Figs. 1 and 2, the cell population may be composed of a plurality of cells contained in one cell capsule. In addition, when the cell capsule has a substantially spherical shape as shown in Fig. 3, the cell population may be composed of an aggregate of a plurality of cells contained in a plurality of substantially spherical cell capsules. In this case, the cell population may be composed of an aggregate of a plurality of cells contained in a plurality of cell capsules simultaneously cultured under the same conditions.

The cell population may be composed of an aggregate of a plurality of cells in a state of being covered with the above cell capsule, or may be composed of an aggregate of a plurality of cells recovered from the above cell capsule.

### [Method for producing cell capsule and cell population]

Hereinafter, a method for producing a cell capsule and a cell population will be described. First, cells and a delivery substance to be contained in the cell capsule are prepared.

The type of cells contained in the core is preferably target cells into which the introduction substance is to be introduced. Such cells may be the above-described cells already listed.

The delivery substance may contain at least one selected from the group consisting of viruses, viral vectors, vaccines, extracellular vesicles, and liposomes, or a complex of these with the introduction substance. The vaccine may be, for example, a virus-like particle and/or an inactivated virus-derived component. Preferably, the delivery substance may be capable of binding to a receptor on the surface of the cells.

The delivery substance may contain an introduction substance to be introduced into the cells. The introduction substance is not particularly limited as long as it is a substance that is to be incorporated into cells. The introduction substance may contain, for example, at least one selected from the group consisting of nucleic acids, proteins, nanoparticles, and drugs. Details of the introduction substance are as described above.

The delivery substance may be electrically neutral in a solvent, or may not be electrically neutral. The semipermeable membrane may be a neutral substance in a solvent, or may be a substance carrying a positive or negative charge. When the semipermeable membrane is made of a substance carrying a positive or negative charge in a solvent, the delivery substance may not be electrically neutral. In this case, the delivery substance is easily confined inside the semipermeable membrane due to electrical interactions. For example, an alginate gel tends to be negatively charged in a solvent. Accordingly, when the semipermeable membrane is made of an alginate gel, a positively charged delivery substance is easily adsorbed onto the alginate gel, and a negatively charged delivery substance is easily confined inside the alginate gel by repulsive force. Since a charged delivery substance is easily confined inside the alginate gel serving as the semipermeable membrane, the size of the delivery substance may be relatively small.

When the delivery substance is a virus or a viral vector, the virus or viral vector may include at least one selected from the group consisting of retroviruses, lentiviruses, adenoviruses, adeno-associated viruses, herpesviruses, and tobacco mosaic viruses. The virus or viral vector may also be a hybrid virus having functions of a plurality of these viruses.

A viral vector is a virus that infects target cells and contains an introduction substance to be introduced into the cells. Such an introduction substance may contain, for example, a nucleic acid encoding at least one selected from the group consisting of receptor proteins, membrane proteins, transcription factors, cytokines, reporter proteins, leukocyte antigens, growth factors, antibodies, and siRNA. The receptor protein is not particularly limited, but may be, for example, chimeric antigen receptor (CAR) or TCR. The reporter protein is not particularly limited, but may be, for example, GFP.

The size of the delivery substance, that is, the maximum diameter, may be, for example, 1000 nm or less, preferably 500 nm or less, and more preferably 200 nm or less. The size of the delivery substance, that is, the maximum diameter, may be, for example, 1 nm or more, preferably 10 nm or more, and more preferably 50 nm or more. The size of the delivery substance may be within a range obtained by arbitrarily combining the above upper limit and lower limit values. This makes it easier for the delivery substance to be confined inside the semipermeable membrane.

The molecular weight of the delivery substance may be, for example, 1 kDa or more, preferably 10 kDa or more, and more preferably 100 kDa or more. The molecular weight of the delivery substance may be, for example, 10,000,000 kDa or less, preferably 1,000,000 kDa or less, and more preferably 500,000 kDa or less. The molecular weight of the delivery substance may be within a range obtained by arbitrarily combining the above upper limit and lower limit values. This makes it easier for the delivery substance to be confined inside the semipermeable membrane.

Next, the cells and the delivery substance are covered with a semipermeable membrane to form a cell capsule, and the cells are cultured in the cell capsule (capsule culture). The configuration of the cell capsule is as described above. Specific examples of methods for forming the cell capsule will be described below. The cells in the cell capsule are cultured in a state of being covered with the semipermeable membrane. The delivery substance contains an introduction substance. The introduction substance contained in the delivery substance is incorporated into the cells along with culturing of the cells. When the delivery substance is a virus or a viral vector, the delivery substance may contain a nucleic acid (introduction substance) encoding a genetic material.

Alternatively and/or additionally, cells into which the introduction substance has been introduced may be cultured in the cell capsule.

The cells in the cell capsule may be cultured in a state in which the delivery substance is bound to a receptor on the surface of the cells, for example, in an infected state.

In capsule culture, the cells in the cell capsule are cultured while the cell capsule is immersed in a medium. The composition and culturing conditions of the medium used in the capsule culture may be any conventionally known ones depending on the cells, the purpose of culture, and the like.

The culturing time is not particularly limited, but may be, for example, 24 hours or more, 36 hours or more, or 48 hours or more. The culturing temperature is not particularly limited, but may be, for example, 4°C or higher, 10°C or higher, or 20°C or higher. The culturing temperature may also be, for example, 40°C or lower. The hydrogen ion index (pH) during culture may be, for example, about 6 to 8. The medium only has to be a solution usable for cell culture, and the composition of the medium may be appropriately set depending on the purpose.

The inventors of the present application have found that by covering cells and a delivery substance with a semipermeable membrane to form a cell capsule and by culturing the cells in the cell capsule, an introduction substance can be efficiently introduced into the cells. This can be presumed to be because the delivery substance is confined together with the cells in a narrow space covered with the semipermeable membrane, thereby making it easier for the introduction substance to be introduced into the cells. In particular, it is considered that the delivery substance is likely to be introduced in the cells by being confined together with the cells in the narrow space covered with the semipermeable membrane.

During culturing of the cells, it is preferable that the volume of the space covered with the semipermeable membrane is 10% or less of the volume of the medium. Accordingly, the cells and the delivery substance are confined in a local region of the medium, that is, in a region inside the cell capsule. This makes it easier for the introduction substance to be further introduced from the delivery substance into the cells.

When the delivery substance is a virus or a viral vector, it is considered that the virus or the viral vector is likely to infect the cells, whereby the efficiency of gene introduction into the cells can be improved.

As one example, by infecting the cells with a virus or a viral vector, it is preferable to form T cells and/or NK cells expressing a chimeric antigen receptor (CAR-T cells and/or CAR-NK cells), T cells expressing a T cell receptor (TCR-T cells), or stem cells, precursor cells, and/or differentiated cells into which other genes have been introduced. Such transformation of the cells is used for the purpose of immune cell therapy. A CAR expression rate can be, for example, 30% or higher, 40% or higher, 50% or higher, 60% or higher, 70% or higher, or 80% or higher. An upper limit of the CAR expression rate is not particularly limited. The inventors also achieved, depending on experimental conditions, a CAR expression rate of about 91%.

In the present embodiment, it is possible to perform gene introduction using a virus or a viral vector without using a coating agent such as RetroNectin that enhances gene transfer efficiency. Even in this case, a high gene introduction efficiency can be maintained. However, it should be noted that the present invention is not limited to not using a coating agent such as RetroNectin, and the coating agent may be used.

In the present embodiment, since the cells are cultured in a state of being accommodated in the cell capsule, it is not necessary to culture the cells on a restricted plane such as a two-dimensional plane. Therefore, scale-up of culture is easy.

The above capsule culture may be culture using feeder cells, or may be feeder-free culture without using feeder cells.

The above capsule culture may be either static culture or dynamic culture. Preferably, the capsule culture can be performed by dynamic culture. That is, the cell capsule may be dynamically cultured in a state of being immersed in a medium.

Since the materials, shapes, sizes, and the like of the semipermeable membrane constituting the cell capsule used in capsule culture are as described above, the description thereof will be omitted.

The cell capsule can be used as it is. For example, the cells in the cell capsule may be further cultured in the form of the cell capsule. In addition, by adding an additive such as a drug into a solvent containing the cell capsule, a response of the cells in the cell capsule to the drug can be examined. Furthermore, a substance produced from the cells can be recovered while the cells are covered with the semipermeable membrane. Moreover, a structure appropriately molded into a desired shape may be formed from the cell capsule. In this case, the cell capsule can be used in a state of the structure.

After capsule culture, the cells may be recovered from the cell capsule. This makes it possible to obtain a cell population containing a plurality of cells into which the introduction substance has been introduced. Recovery of the cells from the cell capsule can be performed, for example, by removing the semipermeable membrane. When the semipermeable membrane contains a hydrogel, such as an alginate gel, as a main component, the semipermeable membrane can be removed by a chemical reaction, an enzymatic reaction, and the like. Specifically, the hydrogel can be removed, for example, by an EDTA/PBS solution or an alginate-degrading enzyme (alginate lyase). The cells can be collected by any known method after removal of the semipermeable membrane.

After capsule culture, a cell product may be recovered from the cell population obtained by the above method. The cell product is not particularly limited as long as it is a substance produced by the cells.

The above cell population can be used, for example, as a cell preparation. In other words, the cell preparation may be obtained from the above cell population. However, the cell preparation may be the above cell population itself, or may be composed of something extracted and/or generated from the above cell population.

When the cell population includes CAR-T cells, CAR-NK cells, and/or TCR-T cells or the like, the cell population can be used, for example, as a cell preparation used for immunotherapy.

### [Formation of cell capsule (1)]

As one example, the cell capsule used in the above capsule culture can be formed as follows. In this example, the semipermeable membrane constituting the cell capsule is a hydrogel.

First, a core, a hydrogel precursor, a gelling solution, and a collection solution are prepared. The core may be a liquid, suspension, or sol containing a delivery substance and cells. The core may contain, for example, a liquid or sol having fluidity, such as a cell suspension. The core may contain, for example, one selected from the group consisting of extracellular matrix, physiological saline, Ringer's solution, medium, culture supernatants, and buffers, or a mixture thereof. The core may further contain, as necessary, the above-described thickener and/or various growth factors.

The core preferably contains a cell suspension, a delivery substance-containing solution, and a thickener. The delivery substance-containing solution is a solution containing the above delivery substance. Here, the delivery substance-containing solution may be a solution containing at least one selected from the group consisting of viruses, viral vectors, vaccines, extracellular vesicles, and liposomes, or a complex of these with the introduction substance. It should be noted that, if necessary, the delivery substance may contain the introduction substance. When the delivery substance is a virus or a viral vector, the core may contain a delivery substance containing a nucleic acid (introduction substance) encoding a genetic material.

Materials constituting the hydrogel precursor are appropriately selected depending on the materials of the hydrogel described above. The hydrogel precursor may include at least one precursor selected from the group consisting of alginate gels, Matrigel, collagen gels, chitosan gels, gelatin, peptide gels, laminin gels, agarose gels, nanocellulose, methylcellulose, dextran, pectin, gellan gum, xanthan gum, guar gum, carrageenan, glucomannan, and fibrin gels.

Preferably, the hydrogel precursor may be a solution mainly composed of an alginate solution (alginate derivative). The alginate solution may be, for example, sodium alginate, potassium alginate, ammonium alginate, or a combination thereof. The alginate derivative may be a natural extract or may be chemically modified. Examples of chemically modified alginates include, for example, methacrylate-modified alginate. The alginate solution is easily crosslinked by a divalent metal ion in a short period of time at room temperature or around room temperature, and tends to form an alginate gel.

The weight of alginate relative to the weight of the solvent in the alginate solution is, for example, 0.1 to 10.0 wt%, preferably 0.25 to 7.0 wt%, and more preferably 0.5 to 5.0 wt%.

The raw material of the alginate gel may preferably be sodium alginate. The M/G ratio of alginate may be, for example, 0.1 to 1.8. The M/G ratio is defined by the composition ratio of D-mannuronic acid and L-guluronic acid in alginates.

The gelling solution may be a solution containing a gelling agent. The gelling agent only has to be a material capable of forming a hydrogel by crosslinking the hydrogel precursor. For example, when the hydrogel precursor is alginate, the gelling agent may be a material capable of providing divalent cations. Examples of such gelling agents include salts of magnesium, calcium, strontium, or barium. Preferably, the gelling agent may be, for example, calcium chloride, calcium carbonate, calcium hydroxide, barium chloride, barium carbonate, or barium hydroxide. That is, the gelling solution may contain, for example, divalent cations such as calcium ions or barium ions.

The concentration of divalent metal ions in the gelling solution may be, for example, 1 mM to 1 M, preferably 20 to 500 mM, and more preferably 100 mM.

The composition of the collection solution is not particularly limited. The collection solution may be, for example, physiological saline or a buffer.

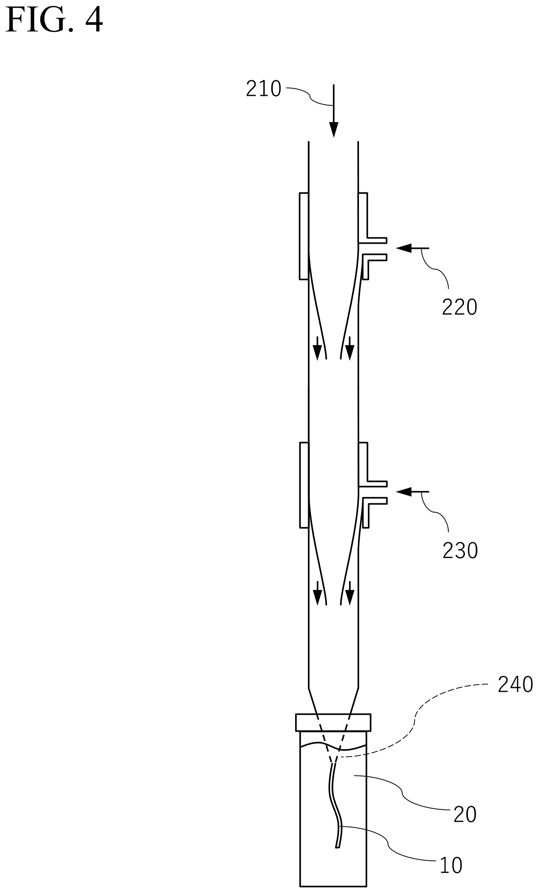

In this example, the formation of the cell capsule includes allowing a core containing cells and a delivery substance and a hydrogel or hydrogel precursor covering the core to continuously flow out into a collection solution. The formation of the cell capsule will be described in more detail with reference to Fig. 4.

Fig. 4 is a view for explaining one example of a method for forming a cell capsule. A device for forming the cell capsule may include a first inlet 210, a second inlet 220, a third inlet 230, and a discharge port 240. The first inlet 210 is an inlet through which the core flows in. The second inlet 220 is an inlet through which the hydrogel precursor flows in. The third inlet 230 is an inlet through which a solution (gelling solution) for gelling the hydrogel precursor flows in.

The hydrogel precursor flows in through the second inlet 220 and joins around the core. As a result, the hydrogel precursor flows along the direction of flow of the core around the core flow. That is, in a cross section perpendicular to the flow of the core, the hydrogel precursor surrounds the core. More specifically, it is preferable that the hydrogel precursor and the core flow so as to form laminar flow.

The gelling solution is a solution for gelling the hydrogel precursor to form a hydrogel. The gelling solution joins the core and the hydrogel precursor on a downstream side of a junction point of the core and the hydrogel precursor. The gelling solution flows along the flow of the hydrogel precursor around the hydrogel precursor surrounding the core. That is, in a cross section perpendicular to the flow of the hydrogel precursor, the gelling solution surrounds the hydrogel precursor. It is preferable that the gelling solution form laminar flow together with the core and the hydrogel precursor.

In this example, the gelling solution merges with the hydrogel precursor, causing the hydrogel precursor to become a hydrogel. The hydrogel precursor may be in a complete hydrogel state when flowing out of the discharge port 240, or may flow out of the discharge port 240 while remaining in the precursor state. Accordingly, the hydrogel or the hydrogel precursor covers the core containing cells and a delivery substance, and flows out into a collection solution 20 together with the core.

In Fig. 4, the discharge port 240 is immersed in the collection solution 20. Alternatively, if possible, the discharge port 240 may be located above the collection solution 20.

In Fig. 4, the hydrogel or the hydrogel precursor continuously flows out into the collection solution together with the core. As a result, a long string-like cell capsule as shown in Figs. 1 and 2 is obtained.

Not limited thereto, the hydrogel or the hydrogel precursor may intermittently flow out into the collection solution together with the core. In this case, the hydrogel or the hydrogel precursor and the core discharged from the discharge port 240 become substantially spherical due to surface tension. As a result, a substantially spherical cell capsule as described with reference to Fig. 3 is obtained.

The cells in the cell capsule are cultured as described above in the cell capsule obtained in this manner.

### [Formation of cell capsule (2)]

As another example, the cell capsule used in the above capsule culture can be formed as follows. In this example, the semipermeable membrane constituting the cell capsule is a hydrogel.

First, a core, a hydrogel precursor, a gelling solution, and a collection solution are prepared. The materials and composition of the core, the hydrogel precursor, the delivery substance, and the introduction substance are as described above. In this example, the collection solution may be a solution for gelling the hydrogel precursor to form a hydrogel. Accordingly, the composition of the collection solution may be the same as the composition of the gelling solution described above.

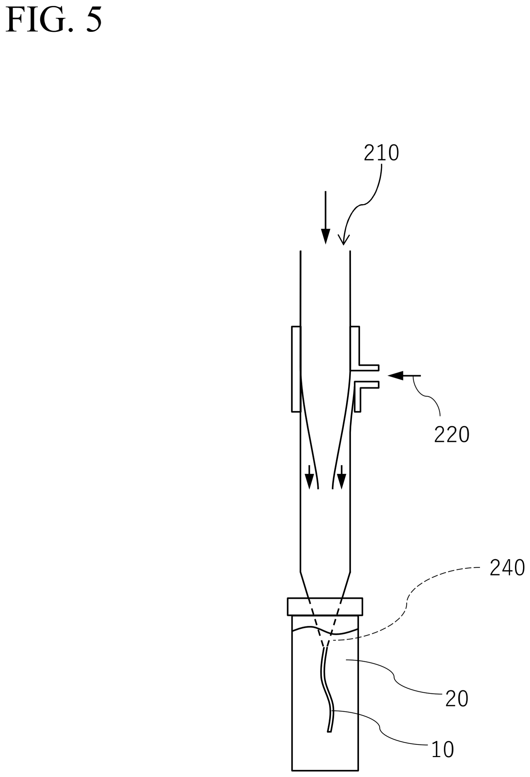

Fig. 5 is a view for explaining another example of a method for forming a cell capsule. In this example, as shown in Fig. 5, a device for forming the cell capsule may include a first inlet 210, a second inlet 220, and a discharge port 240. The first inlet 210 is an inlet through which the core flows in. The second inlet 220 is an inlet through which the hydrogel precursor flows in. The discharge port 240 may be immersed in a collection solution or may be located above the discharge port 240.

The hydrogel precursor flows in through the second inlet 220 and joins around the core. As a result, the hydrogel precursor flows along the direction of flow of the core around the core flow. That is, in a cross section perpendicular to the flow of the core, the hydrogel precursor surrounds the core. More specifically, it is preferable that the hydrogel precursor and the core flow so as to form laminar flow.

The hydrogel precursor covers the core containing cells and a delivery substance, and continuously flows out into the collection solution together with the core. The hydrogel precursor is gelled in the collection solution to form a hydrogel. As a result, a cell capsule having a hydrogel covering the core containing cells and a delivery substance is formed.

In Fig. 5, the hydrogel or the hydrogel precursor continuously flows out into the collection solution together with the core. As a result, a long string-like cell capsule as shown in Figs. 1 and 2 is obtained.

Not limited thereto, the hydrogel or the hydrogel precursor may intermittently flow out into the collection solution together with the core. In this case, the hydrogel or the hydrogel precursor and the core discharged from the discharge port 240 become spherical due to surface tension. As a result, a substantially spherical cell capsule as described with reference to Fig. 3 is obtained.

The cells in the cell capsule are cultured as described above in the cell capsule obtained in this manner.

Several methods for forming a cell capsule have been listed. However, the method for forming a cell capsule is not limited to the above methods. For example, when the core is a gel-like material containing cells and a delivery substance, it is also possible to form a cell capsule by coating the core with a semipermeable membrane composed of a polymer after forming the core containing cells and a delivery substance.

### [Example 1]

Hereinafter, Example 1 will be described in detail. As a preliminary preparation, a construct of Anti-CD19 (FMC-63)-28z was recombined into pMS3 to prepare a plasmid vector, and the plasmid vector was introduced into PlatA cells (Cell Biolabs) using FuGENE (Promega) to prepare a retroviral vector. A solution containing the retroviral vector (virus solution) was previously cryopreserved.

### (Day 0 to Day 3)

Peripheral blood mononuclear cells (PBMCs) were separated from whole blood obtained from a donor using a reagent for human lymphocyte separation, and the PBMCs were suspended in a medium. The reagent for human lymphocyte separation was Ficoll-Paque^{™} (Cytiva). The medium was KBM501 (Kohjin Bio Co., Ltd.) supplemented with 3% inactivated human plasma. In Examples 1 to 3 and Reference Examples 1-1 and 1-2, the "medium" used below is also of the same composition. Next, "ImmunoCult Human CD3/CD28/CD2 T Cell Activator (STEMCELL Technologies)" was added to the medium, and culturing of the PBMCs was started.

### (Day 4)

On the fourth day (Day 4) from the start of culturing, PBMCs were recovered from the medium, and the number of cells was counted. The above retrovirus and the PBMCs recovered from the medium were encapsulated according to the method described in "Formation of cell capsule (1)" above.

Specifically, first, a core, a hydrogel precursor, a gelling solution, and a collection solution were prepared. The core was adjusted by suspending a virus solution containing a retrovirus (delivery substance) and cells recovered on Day 4 at 2 × 10⁵ cells/well in a medium supplemented with 0.6 w/v% methylcellulose. The amount of virus solution used was one-eighth of a concentrated retroviral vector (10 mL of supernatant that had produced the viral vector).

The hydrogel precursor was a sodium alginate solution. The sodium alginate solution was produced by adding sodium alginate ("High-G ALG300" manufactured by Kimica Corporation) to physiological saline and stirring. The concentration of sodium alginate relative to the physiological saline was 1.0 w/v%.

The gelling solution was an aqueous solution containing 100 mM calcium chloride and 3 w/v% sucrose. The collection solution was physiological saline.

Using the core, the hydrogel precursor, and the gelling solution, a cell capsule was produced based on the method described in "Formation of cell capsule (1)" (see Fig. 4). That is, a flow of the core, a flow of the hydrogel precursor around the flow of the core, and a flow of the gelling solution around the flow of the hydrogel precursor were formed, and these flows were discharged into the collection solution.

The hydrogel precursor was crosslinked by contact with the gelling solution to form a substantially tubular alginate gel. As a result, a long string-like cell capsule as shown in Figs. 1 and 2 was formed in the collection solution. The inner diameter of the alginate gel (semipermeable membrane) constituting the cell capsule was 150 to 250 µm, and the outer diameter of the alginate gel (semipermeable membrane) was 300 to 450 µm.

The cell capsule was taken out of the collection solution, placed in each well of a 6-well plate together with 1.5 mL of medium, and static culture of the cells was started in a 5% CO₂ incubator at a temperature of 37°C.

### (Day 5 to Day 11)

On Day 6, 1.5 mL of medium was added. On Day 8, 3 mL of medium was added. On Day 10, 6 mL of medium was added.

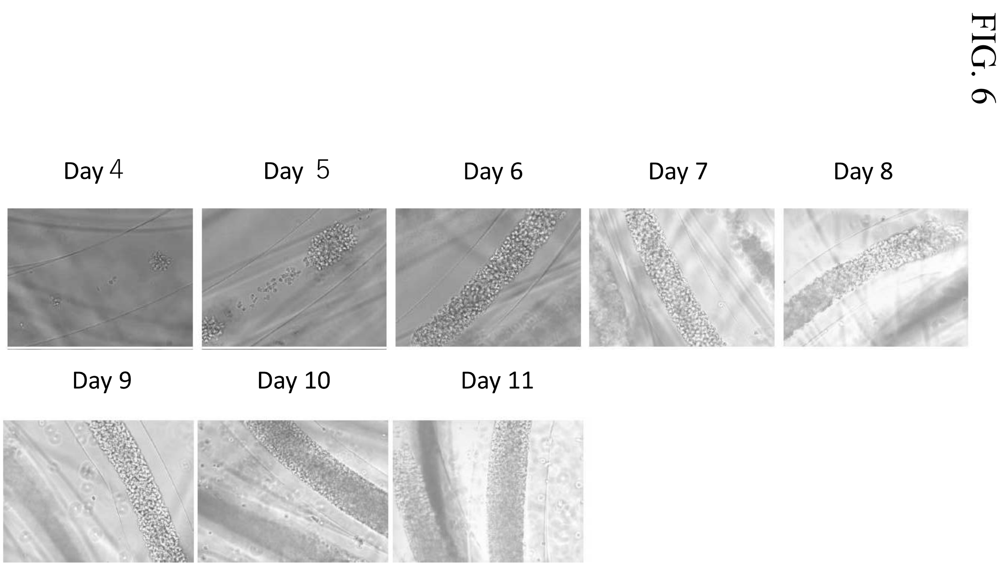

Fig. 6 is a view showing microscopic photographs of cell capsules from Day 4 to Day 11 in Example 1. In Fig. 6, a state in which cells are cultured in the cell capsules is shown.

On Day 11, the cells were recovered. Specifically, a stock solution of alginate lyase (Nagase ChemteX Corporation) at a concentration of 0.1% was added to the medium, allowed to stand at 37°C for about 10 minutes, and the cells were dispersed and recovered by pipetting. The stock solution was prepared by dissolving alginate lyase in PBS to 8% and filtering with a 0.22 µm filter.

### [Reference Example 1-1]

Reference Example 1-1 will be described below. Also in Reference Example 1-1, the same virus and cells as in Example 1 were used.

The culturing of cells in Reference Example 1-1 was carried out in the same manner as in Example 1, except that the cells were cultured without using a cell capsule. Specifically, in Reference Example 1-1, on Day 4, PBMCs recovered from the medium were seeded in a 24-well plate coated with a retrovirus and RetroNectin.

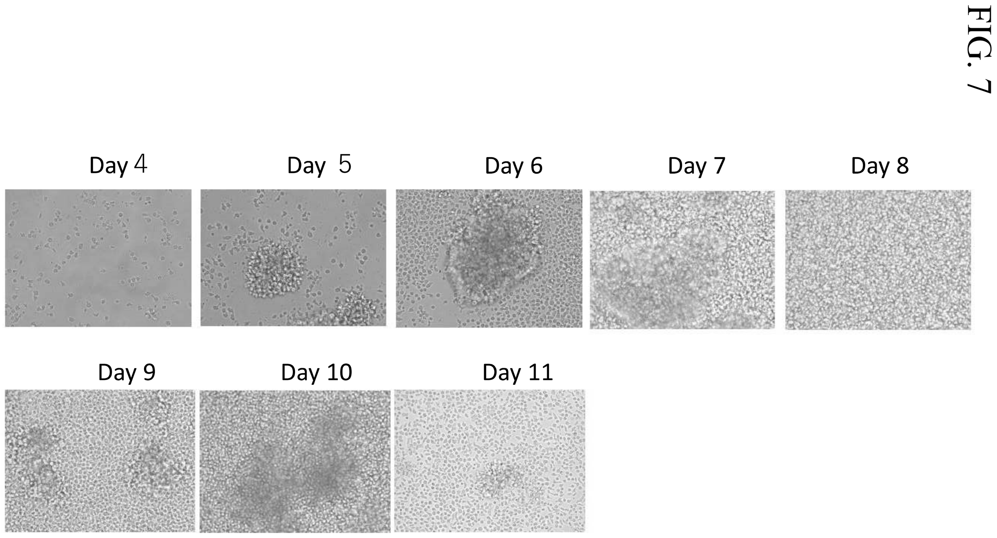

On Day 6, 1.5 mL of medium was added. On Day 8, 3 mL of medium was added. On Day 10, 6 mL of medium was added. Fig. 7 is a view showing microscopic photographs of cell capsules from Day 4 to Day 11 in Reference Example 1-1.

In Reference Example 1-1, on Day 11, the cells were dispersed and recovered by pipetting.

### [Reference Example 1-2]

Reference Example 1-2 will be described below. The culturing of cells in Reference Example 1-2 was carried out in the same manner as in Reference Example 1-1, except that a retrovirus was not used.

### [Analysis of cell population]

For the cell populations obtained in Example 1 and Reference Examples 1-1 and 1-2, CAR expression was measured using a flow cytometer (BD LSRFortessa^{™} X-20). After reaction with an anti-human k antibody (rabbit IgG) (MBL), a reaction with Alexa 488-labeled anti-rabbit IgG (goat polyclonal) (Invitrogen) was performed, and further staining with APC/Cy7-labeled anti-human CD8 (BioLegend) and APC-labeled anti-human CD4 antibody (BioLegend) was added, followed by measurement with the flow cytometer. CAR molecule expression rates in each of the CD4 and CD8 fractions were calculated (recognized by the anti-human k antibody).

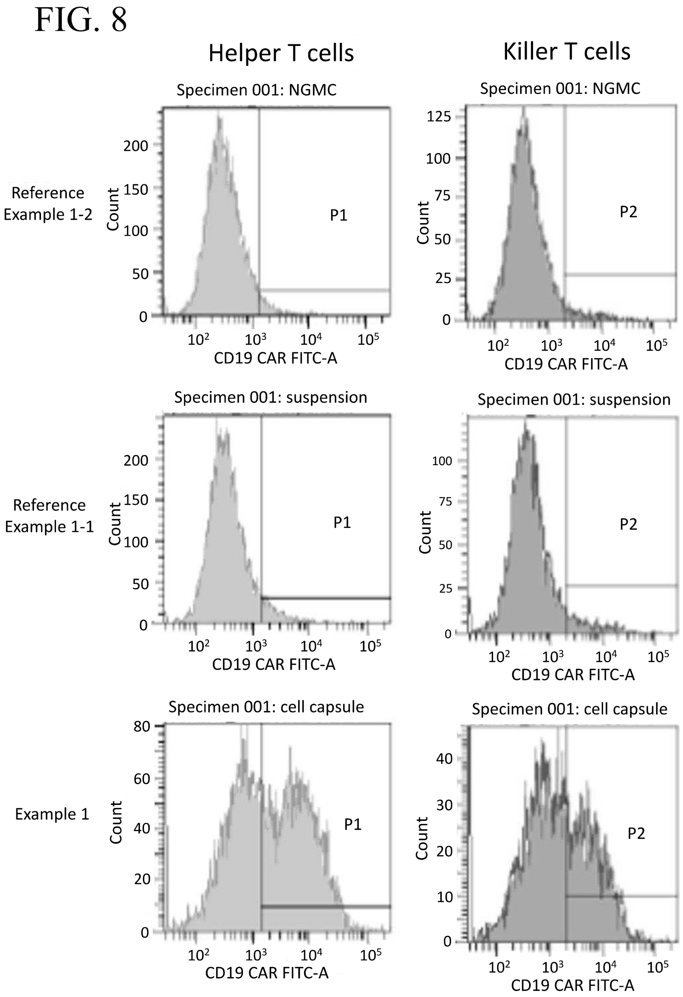

Fig. 8 is a view showing analysis results of cell populations by flow cytometry in Example 1 and Reference Examples 1-1 and 1-2. The vertical axis in Fig. 8 represents the number of cells. The horizontal axis in Fig. 8 represents the fluorescence intensity of CD19 CAR and indicates the level of CAR expression. In the graphs of Fig. 8, the right side of the vertical line represents the cell population (positive population) expressing CAR, and the left side of the vertical line represents the cell population (negative population) not expressing CAR.

In addition, Table 1 below shows CAR molecule expression rates (positivity rates) for the cell populations in Example 1 and Reference Examples 1-1 and 1-2. Table 1 shows CAR expression rates in the CD4-positive cell population (helper T cells) and in the CD8-positive cell population (killer T cells). The helper T cells express CD4 and do not express CD8. The killer T cells express CD8 and do not express CD4.

**[Table 1]**

| | Helper T cells | Killer T cells |
|---|---|---|
| Example 1 | 47.5% | 39.4% |
| Reference Example 1-1 | 4.9% | 7.7% |
| Reference Example 1-2 | 3.2% | 6.7% |

From Fig. 8 and Table 1, it can be seen that the cell population in Example 1 had higher CAR molecule expression rates in both helper T cells and killer T cells than the cell populations in Reference Examples 1-1 and 1-2. This indicates that culturing the cells in the cell capsule in which the cells and the virus were covered with a semipermeable membrane improves infection efficiency.

### [Example 2]

Hereinafter, Example 2 will be described in detail.

### (Day 0 to Day 2)

On Day 0 to Day 2, acquisition and culturing of peripheral blood mononuclear cells (PBMCs) were carried out in the same manner as in Example 1.

### (Day 3)

In Example 2, on Day 3, PBMCs were recovered from the medium, and the thawed retrovirus and the PBMCs recovered from the medium were encapsulated according to the method described in "Formation of cell capsule (1)" above. Formation of the cell capsule and culturing of the cells were carried out under the same conditions and by the same methods as those carried out on Day 4 in Example 1.

### (Day 4 to Day 10)

On Day 5, 1.5 mL of medium was added. On Day 7, 3 mL of medium was added. On Day 9, 6 mL of medium was added. On Day 10, the cells were recovered. Recovery of the cells was carried out in the same manner as in the method carried out on Day 11 in Example 1.

### [Reference Example 2-1]

Reference Example 2-1 will be described below. Also in Reference Example 2-1, the same virus and cells as in Example 2 were used.

The culturing of cells in Reference Example 2-1 was carried out in the same manner as in Example 2, except that the cells were cultured without using a cell capsule. Specifically, in Reference Example 2-1, on Day 3, PBMCs recovered from the medium were seeded in a 24-well plate coated with a retrovirus and RetroNectin.

On Day 5, 1.5 mL of medium was added. On Day 7, 3 mL of medium was added. On Day 9, 6 mL of medium was added. In Reference Example 2-1, on Day 10, the cells were dispersed and recovered by pipetting.

### [Reference Example 2-2]

Reference Example 2-2 will be described below. The culturing of cells in Reference Example 2-2 was carried out in the same manner as in Reference Example 2-1, except that a retrovirus was not used.

### [Analysis of cell population]

For the cell populations obtained in Example 2 and Reference Examples 2-1 and 2-2, CAR molecule expression rates in each of the CD4 and CD8 fractions were calculated in the same manner as in Example 1 (recognized by the anti-human k antibody).

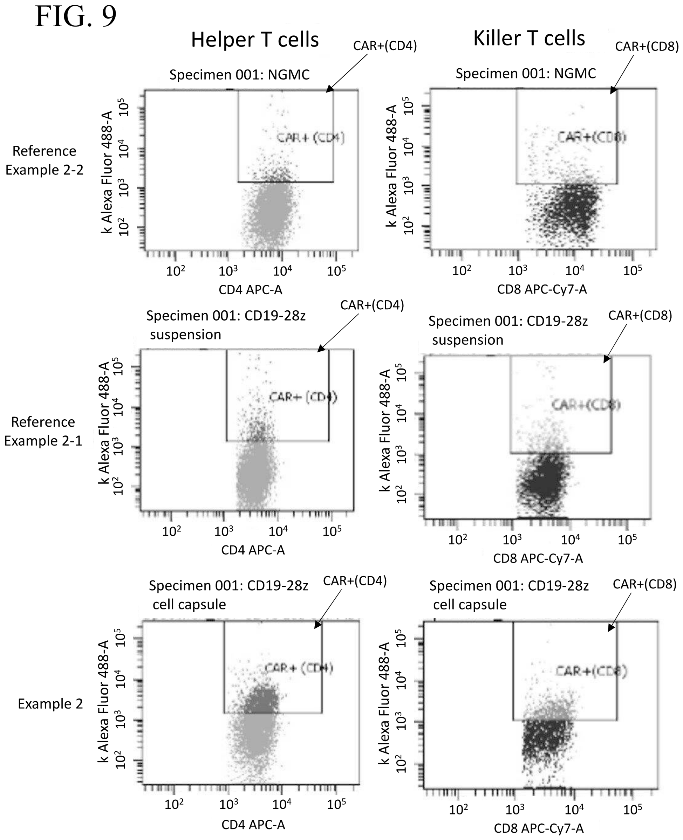

Fig. 9 is a view showing analysis results of cell populations by flow cytometry in Example 2 and Reference Examples 2-1 and 2-2. The longitudinal axis in Fig. 9 represents the fluorescence intensity of CD19 CAR and indicates the level of CAR expression. The horizontal axis in Fig. 9 represents markers of helper T cells (CD4) and killer T cells (CD8). In the graphs shown in Fig. 9, the dots plotted within the squares represent helper T cells (CD4) and killer T cells (CD8) expressing CAR.

In addition, Table 2 below shows CAR molecule expression rates (positivity rates) for the cell populations in Example 2 and Reference Examples 2-1 and 2-2. Table 2 shows CAR expression rates in the CD4-positive cell population (helper T cells) and in the CD8-positive cell population (killer T cells). That is, the proportion of dots plotted within the squares in the graphs shown in Fig. 9 corresponds to the CAR expression rates shown in Table 2.

**[Table 2]**

| | Helper T cells | Killer T cells |
|---|---|---|
| Example 2 | 24.5% | 23.4% |
| Reference Example 2-1 | 4.7% | 7.6% |
| Reference Example 2-2 | 2.8% | 8.4% |

From Fig. 9 and Table 2, it can be seen that the cell population in Example 2 had higher CAR molecule expression rates in both helper T cells and killer T cells than the cell populations in Reference Examples 2-1 and 2-2. This indicates that culturing the cells in the cell capsule in which the cells and the virus were covered with a semipermeable membrane improves infection efficiency.

For the cell populations in Example 2 and Reference Example 2-1, the amount of cytokine (IFN-γ) produced was measured. Specifically, the cell populations recovered on Day 10 in Example 2 and Reference Example 2-1 were cultured for 20 hours on plates coated with Recombinant Human CD19 Fc Chimera Protein (R&D) (2 mg/mL). Thereafter, the supernatants were recovered, and quantitative determination of g-interferon was performed using a Human IFN Gamma ELISA Kit (Invitrogen).

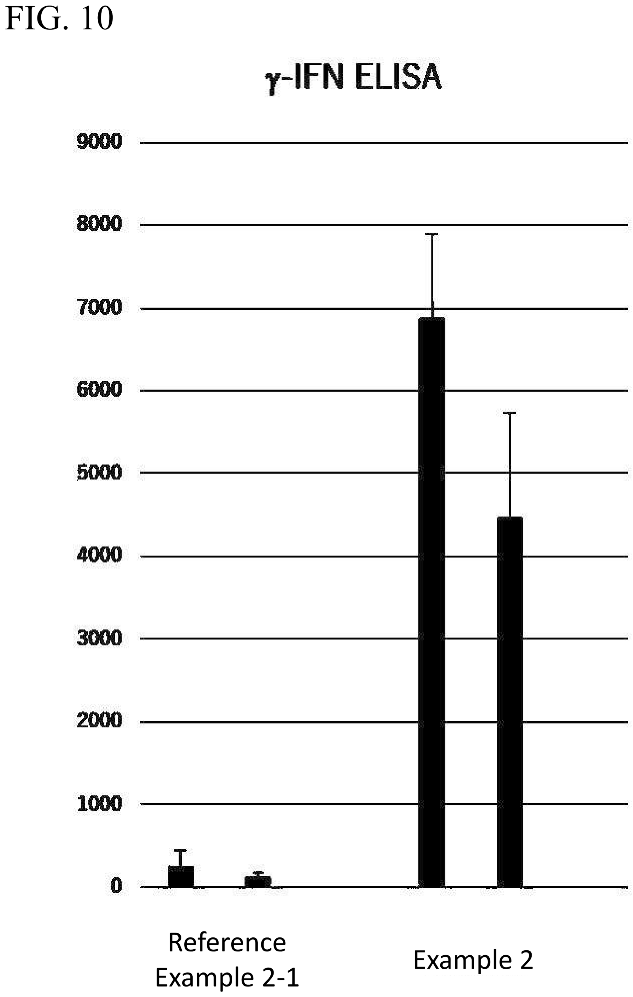

Fig. 10 is a view showing results of measurement of the amount of cytokine (IFN-γ) produced from the cell populations in Example 2 and Reference Example 2-1. In Fig. 10, two cell populations were respectively prepared by the methods described in Example 2 and Reference Example 2-1, and measurement results for the two cell populations are shown. From Fig. 10, it can be seen that the cell population in Example 2 contained many T cells expressing a chimeric antigen receptor, and thus the amount of IFN-γ produced was large. On the other hand, since the cell population in Reference Example 2-1 was not significantly infected with the virus, the amount of IFN-γ produced was low.

### [Example 3]

Hereinafter, Example 3 will be described in detail. As a preliminary preparation, a construct of Anti-CD19 (FMC-63)-28z was recombined into pMS3 to prepare a plasmid vector, and the plasmid vector was introduced into PlatA cells (Cell Biolabs) using FuGENE (Promega) to prepare a retrovirus. A solution containing the retroviral vector (virus solution) was previously cryopreserved.

### (Day 0 to Day 2)

On Day 0 to Day 2, acquisition and culturing of peripheral blood mononuclear cells (PBMCs) were carried out in the same manner as in Example 1.

### (Day 3)

In Example 3, on Day 3, PBMCs recovered from the medium were seeded at 2 × 10⁵ cells/well in a 24-well plate coated with a thawed retrovirus and RetroNectin.

### (Day 4)

In Example 3, the PBMCs seeded in the 24-well plate on Day 3 were recovered, and on Day 4, the recovered PBMCs were encapsulated together with the retrovirus according to the method described in "Formation of cell capsule (1)." The conditions and method of encapsulation were the same as those in Example 1.

### (Day 5 to Day 11)

From Day 5 to Day 11, the procedure was the same as in Example 1. Accordingly, on Day 6, 1.5 mL of medium was added. On Day 8, 3 mL of medium was added. On Day 10, 6 mL of medium was added. On Day 11, the cells were recovered. Recovery of the cells was carried out in the same manner as in Example 1.

### [Reference Example 3-1]

Reference Example 3-1 will be described below. Also in Reference Example 3-1, the same virus and cells as in Example 3 were used.

The culturing of cells in Reference Example 3-1 was carried out in the same manner as in Example 3, except that the cells were cultured without using a cell capsule. Specifically, in Reference Example 3-1, on Day 4, PBMCs recovered from the medium were seeded in a 24-well plate coated with a retrovirus and RetroNectin without encapsulation.

On Day 6, 1.5 mL of medium was added. On Day 8, 3 mL of medium was added. On Day 10, 6 mL of medium was added. In Reference Example 3-1, on Day 11, the cells were dispersed and recovered by pipetting.

### [Reference Example 3-2]

Reference Example 3-2 will be described below. The culturing of cells in Reference Example 3-2 was carried out in the same manner as in Reference Example 3-1, except that a retrovirus was not used.

### [Analysis of cell population]

For the cell populations obtained in Example 3 and Reference Examples 3-1 and 3-2, CAR molecule expression rates in each of the CD4 and CD8 fractions were calculated in the same manner as in Example 1 (recognized by the antihuman k antibody).

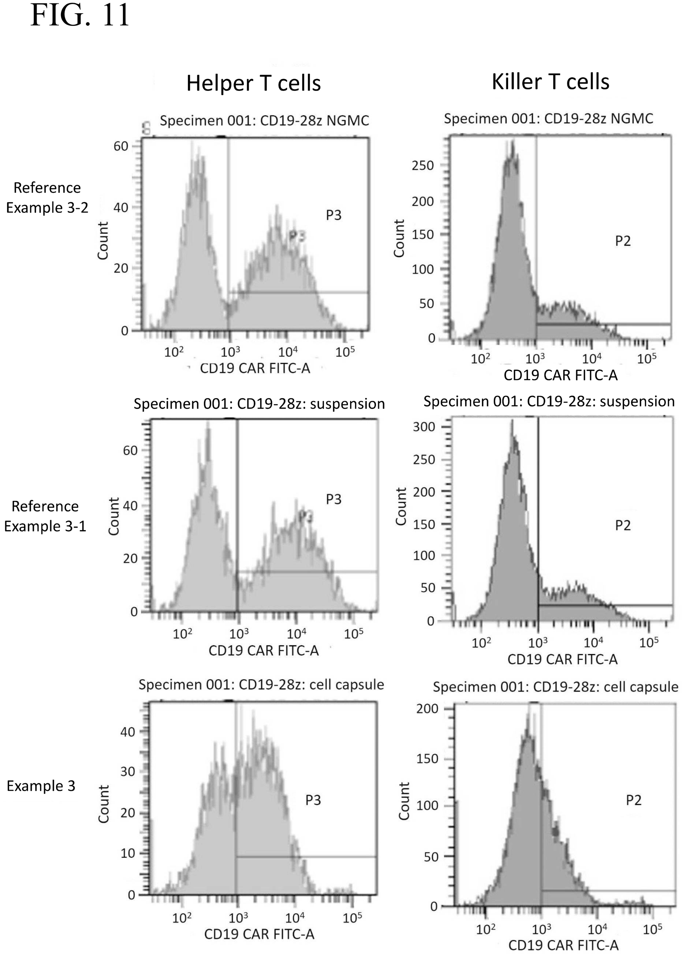

Fig. 11 is a view showing analysis results of cell populations by flow cytometry in Example 3 and Reference Examples 3-1 and 3-2. The vertical axis in Fig. 11 represents the number of cells. The horizontal axis in Fig. 11 represents the fluorescence intensity of CD19 CAR and indicates the level of CAR expression. In the graphs of Fig. 11, the right side of the vertical line represents the cell population (positive population) expressing CAR, and the left side of the vertical line represents the cell population (negative population) not expressing CAR.

In addition, Table 3 below shows CAR molecule expression rates (positivity rates) for the cell populations in Example 3 and Reference Examples 3-1 and 3-2. Table 3 shows CAR expression rates in the CD4-positive cell population (helper T cells) and in the CD8-positive cell population (killer T cells).

**[Table 3]**

| | Helper T cells | Killer T cells |
|---|---|---|
| Example 3 | 58.8% | 32.1% |
| Reference Example 3-1 | 52.6% | 26.4% |
| Reference Example 3-2 | 52.6% | 24.8% |

From Fig. 11 and Table 3, it can be seen that the cell population in Example 3 had higher CAR molecule expression rates in both helper T cells and killer T cells than the cell populations in Reference Examples 3-1 and 3-2. This indicates that culturing the cells in the cell capsule in which the cells and the virus were covered with a semipermeable membrane improves infection efficiency.

### [Example 4]

Hereinafter, Example 4 will be described in detail.

### (Preliminary preparation)

Cells were seeded in a medium at 1 × 10⁶ cells/10 cm dish. The cells were "AAV-293 Cells (Agilent; Product No. 240073)." The medium was "DMEM, high glucose, pyruvate (Gibco; Product No. 11995040)" supplemented with 10% fetal bovine serum (FBS).

### (Day 0)

A cell capsule was produced by the following procedure. Formation of the cell capsule was carried out according to the method described in "Formation of cell capsule (1)" above. In the formation of the cell capsule, first, a core, a hydrogel precursor, a gelling solution, and a collection solution were prepared.

The core was prepared by the following procedure.
(1) Plasmids of AAV2, Helper, and GFP were mixed.
(2) A transfection solution containing the mixed plasmids was prepared using a reagent AAV-MAX.
(3) Preparation of NT solution mixture: the transfection solution was diluted with medium (DMEM (without FBS)) (25 ng/µL).
(4) Cells were detached from a preliminarily prepared 10 cm dish and dispensed into tubes at 6 × 10⁶ cells/tube.
(5) The tubes were centrifuged to remove the medium, and the transfection solution was added to the tubes at 240 uL/tube. Here, the amount of plasmids in each tube was 6 µg/tube.
(6) Methylcellulose was added to the tubes such that the final volume was 1200 µL and the final concentration of methylcellulose was 0.3%. The solution thus obtained was used as the core.

The hydrogel precursor was a sodium alginate solution. The sodium alginate solution was produced by adding sodium alginate ("High-G ALG300" manufactured by Kimica Corporation) to physiological saline and stirring. The concentration of sodium alginate relative to the physiological saline was 1.0 w/v%.

The gelling solution was an aqueous solution containing 100 mM calcium chloride and 3 w/v% sucrose. The collection solution was physiological saline.

Using the core, hydrogel precursor, and gelling solution described above, a cell capsule was produced based on the method described in "Formation of cell capsule (1)" (see Fig. 4). That is, a flow of the core, a flow of the hydrogel precursor around the flow of the core, and a flow of the gelling solution around the flow of the hydrogel precursor were formed, and these flows were discharged into the collection solution.

The hydrogel precursor was crosslinked by contact with the gelling solution to form a substantially tubular alginate gel. As a result, a long string-like cell capsule as shown in Figs. 1 and 2 was formed in the collection solution.

The cell capsule was taken out of the collection solution, placed in each well of a 6-well plate together with 4.0 mL of medium, and culturing of the cells was started at a temperature of 37°C.

### (Day 3)

On Day 3, the cells were recovered and GFP-positive cells were confirmed. Confirmation of the GFP-positive cells was performed by observation with a fluorescence microscope. An AAV vector was extracted from the cells and stored at -80°C. In the extraction of the AAV vector, first, the cell capsule was placed in a 10mM EDTA/PBS solution to dissolve the alginate gel. The solution in which the alginate gel was dissolved was centrifuged, and the supernatant was removed. Thereafter, the AAV vector was extracted using "AAVpro Titration Kit (for Real Time PCR) Ver. 2 (Takara Bio Inc.)."

### [Reference Example 4-1]

Reference Example 4-1 will be described below.

### (Preliminary preparation)

Cells were seeded at 4.5 × 10⁵ cells/10 cm dish. The type of cells and the medium were the same as those in Example 4.

### (Day 0)

An NT solution and a transfection solution were prepared in the same manner as in Example 1. For the preliminarily prepared 10 cm dish, the medium was replaced, and the transfection solution and the NT solution were added at 36 µL/well. Culturing of the cells was then continued.

### (Day 3)

On Day 3, the cells were recovered and GFP-positive cells were confirmed. Confirmation of the GFP-positive cells was performed by observation with a fluorescence microscope. An AAV vector was extracted from the cells and stored at -80°C. In the extraction of the AAV vector, first, a 500 mM EDTA solution was added to the culture supernatant so that the final concentration became 6.25 mM. After 10 minutes, the detached cells were recovered and centrifuged, and then the supernatant was removed. Thereafter, the AAV vector was extracted using "AAVpro Titration Kit (for Real Time PCR) Ver. 2 (Takara Bio Inc.) ."

### [Reference Example 4-2]

Reference Example 4-2 will be described below. In Reference Example 4-2, the procedure was carried out in the same manner as in Reference Example 4-1, except that the NT solution and the transfection solution were not added on Day 0.

### (Day 3)

On Day 3, cells were recovered and GFP-positive cells were confirmed. An AAV vector was extracted from the cells and stored at -80°C. Confirmation of the GFP-positive cells and extraction of the AAV vector were carried out in the same manner as in Reference Example 4-1.

### [Analysis of cell population]

For the cell populations obtained in Example 4 and Reference Examples 4-1 and 4-2, AAV vector genomes were extracted using "AAVpro Titration Kit (for Real Time PCR) Ver. 2 (Takara Bio Inc.)." Based on the extracted AAV vector genomes, qPCR was performed by a probe method. The qPCR conditions were as follows.
(Initial denaturation) 1 Cycle:
   95°C/30 seconds
(PCR reaction) 40 Cycles:
   95°C/3 seconds
   50°C/34 seconds

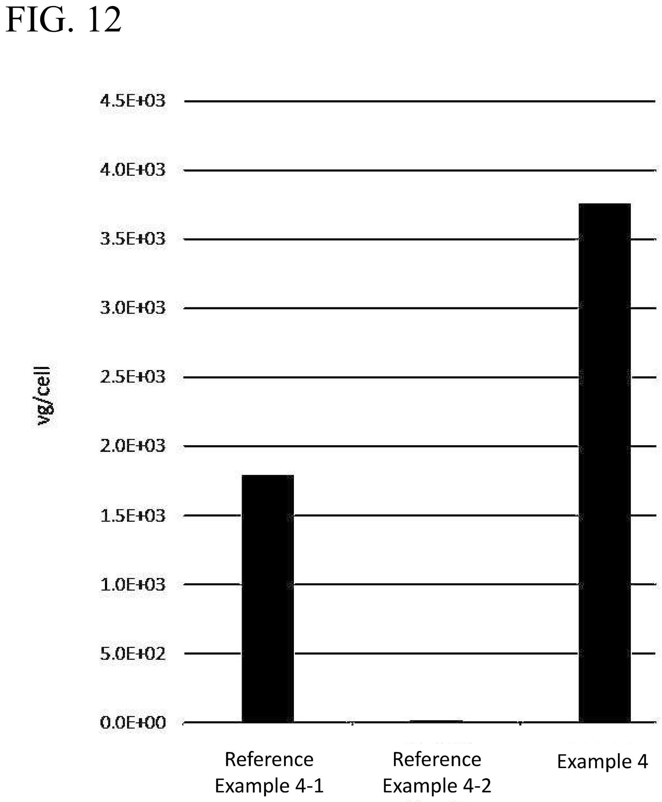

Fig. 12 is a view showing analysis results of cell populations in Example 4 and Reference Examples 4-1 and 4-2. The vertical axis in Fig. 12 represents a value obtained by dividing the number of vector genomes by the number of cells (vg/cell). Accordingly, the vertical axis corresponds to the number of AAV vectors produced per cell.

From Fig. 12, it can be said that by performing transfection using the cell fiber, more AAVs were produced in the cell population in Example 4.

As described above, the contents of the present invention have been disclosed through the embodiments, but it should not be understood that the description and the drawings constituting a part of the disclosure limit the present invention. From this disclosure, various alternative embodiments, examples, and operational techniques will become apparent to those skilled in the art. Therefore, the technical scope of the present invention is defined only by the matters specifying the invention according to the claims appropriate from the above description.

This application claims priority based on Japanese Patent Application No. 2023-075061 filed on April 28, 2023, the entire contents of which are incorporated herein by reference.

## Claims

1. A method for producing a cell population, comprising:
covering cells and a delivery substance with a semipermeable membrane to form a cell capsule; and
culturing the cells in the cell capsule.

2. The method for producing a cell population according to claim 1, comprising: incorporating an introduction substance into the cells by the delivery substance in the cell capsule.

3. The method for producing a cell population according to claim 2, wherein the introduction substance includes at least one selected from a group consisting of nucleic acid, protein, nanoparticle, and drug.

4. The method for producing a cell population according to any one of claims 1 to 3, wherein the delivery substance includes a virus or a viral vector, or a complex of these with the introduction substance.

5. The method for producing a cell population according to any one of claims 1 to 3, wherein the delivery substance includes at least one selected from a group consisting of vaccine, extracellular vesicle and liposome, or includes a complex of these with the introduction substance.

6. The method for producing a cell population according to any one of claims 1 to 5, wherein the semipermeable membrane includes a hydrogel.

7. The method for producing a cell population according to claim 6, wherein the hydrogel includes an alginate gel.

8. The method for producing a cell population according to any one of claims 1 to 7, wherein
the semipermeable membrane has a substantially tubular shape or a substantially spherical shell shape, and
an inner diameter of the semipermeable membrane is in a range of 20 µm to 4000 µm.

9. The method for producing a cell population according to any one of claims 1 to 8, wherein
the cells are cultured with the cell capsule being immersed in a medium, and
a volume of a space covered with the semipermeable membrane is 10% or less of a volume of the medium.

10. The method for producing a cell population according to any one of claims 1 to 9, further comprising: recovering the cells from the cell capsule after the culturing.

11. The method for producing a cell population according to any one of claims 1 to 4 and 6 to 10, wherein
the delivery substance includes a virus or a viral vector, or a complex of these with the introduction substance, and
the method includes infecting the cells with the virus or viral vector by the culturing of the cells.

12. The method for producing a cell population according to any one of claims 1 to 4 and 6 to 10, wherein
the delivery substance includes a virus or a viral vector, or a complex of these with the introduction substance,
the introduction substance includes a nucleic acid encoding a chimeric antigen receptor,
the cells are at least one selected from blood cells, T cells, NK cells, dendritic cells, hematopoietic stem cells, lymphocyte precursor cells, and hematopoietic precursor cells, and
the culturing of the cells forms T cells expressing a chimeric antigen receptor or T cells expressing a T cell receptor.

13. A cell population obtained by the method for producing a cell population according to any one of claims 1 to 12.

14. A cell preparation obtained from the cell population according to claim 13.

15. A cell product obtained from the cell population according to claim 13.

16. A cell capsule comprising:
a core containing cells and an introduction substance; and
a semipermeable membrane covering the core.

17. The cell capsule according to claim 16, wherein the introduction substance contains at least one selected from a group consisting of nucleic acid, protein, nanoparticles, and drug.

18. The cell capsule according to claim 16 or 17, wherein the core contains a plurality of cells, and
some of the plurality of cells incorporate the introduction substance.

19. The cell capsule according to any one of claims 16 to 18, wherein the core contains a delivery substance.

20. The cell capsule according to claim 19, wherein the delivery substance contains a virus or a viral vector, or a complex of these with the introduction substance.

21. The cell capsule according to claim 19, wherein the delivery substance contains at least one selected from a group consisting of vaccine, extracellular vesicle and liposome, or a complex of these with the introduction substance.

22. The cell capsule according to any one of claims 16 to 21, wherein the semipermeable membrane contains a hydrogel.

23. The cell capsule according to claim 22, wherein the hydrogel contains an alginate gel.

24. The cell capsule according to any one of claims 16 to 23, wherein
the semipermeable membrane has a substantially tubular shape or a substantially spherical shell shape, and
an inner diameter of the semipermeable membrane is in a range of 20 µm to 4000 µm.
